# EUROPÄISCHE PATENTANMELDUNG

(11) **EP 0 624 799 A2**
(43) Veröffentlichungstag der Anmeldung: **17.11.1994**
(21) Anmeldenummer: 94250112.3
(22) Anmeldetag: 29.04.1994
(51) Int. Cl.: G01N 33/18, E21B 7/00, E21B 7/26, E21B 49/08, G01N 1/10, G01N 33/24, G01N 21/64

(54) **Drucksonde zum quantitativen Nachweis von im Grundwasser vorhandenen Schadstoffen**

(30) Priorität: 14.05.1993 DE 4316690
(71) Anmelder: DANKWART KLEIN ERDBOHRUNGEN GmbH & Co., BRUNNENBAU KG, D-13507 Berlin (DE)
(72) Erfinder: Geisen, Gerhard, D-13507 Berlin (DE)
(74) Vertreter: Wablat, Wolfgang, Dr.Dr.

(57) **Zusammenfassung**

Die Erfindung beschreibt eine Drucksonde (1) zum quantitativen Nachweis von im Grundwasser vorhandenen Schadstoffen, insbesondere von Mineralölkohlenwasserstoffen (MKW) und/oder polycyclischen aromatischen Kohlenwasserstoffen (PAK), an Ort und Stelle mit einer Meßeinrichtung (6).

Der Erfindung liegt die Aufgabe zugrunde, eine Drucksonde zu schaffen, mit der eine unmittelbare vor Ort (in situ) vorzunehmende Aussage über eventuelle Schadstoffgehalte in einer entsprechenden erforderlichen Tiefensondierung vorgenommen und ausgewertet werden kann. Gelöst wird diese Aufgabe durch die Kombination folgender technischer Merkmale, daß
a) die Drucksonde (1) zur Ermittlung bodenphysikalischer Parameter einen zusätzlichen Meßkopf der ein Meßkammer (9) zur Aufnahme einer zu messenden Grundwasserprobe und ein an die Meßeinrichtung (6) angeschlossenes Lichtleitkabel (12) aufweist, und
b) die Meßeinrichtung vorzugsweise aus einer Lasertechnik (14) mit einem angeschlossenen Rechner (17) zur Auswertung besteht, wobei als Meßverfahren die zeitauflösende laserinduzierte Fluoreszenz eingesetzt wird.

## Beschreibung

Die Erfindung betrifft eine Drucksonde zum quantitativen Nachweis von im Grundwasser vorhandenen Schadstoffen der im Oberbegriff des Patentanspruchs 1 angegebenen Gattung.

Es ist eine Probeentnahme-Einrichtung für Voruntersuchungen möglicherweise kontaminierter Bodenschichten bekannt, bei der zur Entnahme fluider Proben, wie Grundwasser, ein Probennehmer als Sondenprojektil mittels einer Beschleunigungseinrichtung in die Probenumgebung eingeschossen wird (DE 4024214 A1). Die entnommene Probe muß dann in ein Labor zur Untersuchung transportiert werden. Das Untersuchungsergebnis kann nicht sofort an Ort und Stelle festgestellt werden. Dies ist ein offensichtlicher Nachteil.

Der Erfindung liegt deshalb die Aufgabe zugrunde, eine Drucksonde zum Nachweis von im Grundwasser verschleppten Schadstoffen zu schaffen, mit der eine unmittelbare vor Ort (in situ) vorzunehmende Aussage über eventuelle Schadstoffgehalte vorgenommen werden kann.

Diese Aufgabe wird gemäß der Erfindung durch die im kennzeichnenden Teil des Patentanspruchs 1 angegebenen Merkmale gelöst.

Vorteilhafte Weiterentwicklungen sind in den abhängigen Ansprüchen gekennzeichnet.

Die mit der Erfindung erzielten Vorteile bestehen insbesondere darin, daß innerhalb kurzer Zeit und ohne Bohrungen zur Förderung von Meßproben aus der Tiefe des Bodens, ein Boden-Standort in seiner horizontalen und vertikalen Ausbreitung in fast jeder Tiefe untersucht und ein entsprechendes Meßergebnis vor Ort festgestellt werden kann.

Die Erfindung wird anhand von in der Zeichnung dargestellten Ausführungsbeispielen näher erläutert. Es zeigt
- Fig. 1: eine Seitenansicht (teilweise geschnitten) eines ersten Ausführungsbeispiels der erfindungsgemäßen Drucksonde mit einem als Meßkammer ausgebildeten Meßkopf, wobei in der Kammer die Meßprobe aufgenommen wird,
- Fig. 2: eine Seitenansicht des unteren Endes der Drucksonde nach einem zweiten Ausführungsbeispiel mit zwei in der Meßkammer angeordneten Öffnungen, die ein Durchströmen der Meßkammer mit dem zu unterscheidenden Grundwasser ermöglichen,
- Fig. 2a: eine Seitenansicht des unteren Endes der Drucksonde nach einem dritten Ausführungsbeispiel mit einer in der Meßkammer angeordneten Öffnung, in die ein durchsichtiger Kristall angeordnet ist, welcher für den Laserstrahl durchlässig ist, so daß das außen an der Meßkammer befindliche Medium gemessen werden kann,
- Fig. 3: einen Längsschnitt der als Fahrzeug ausgebildeten Meßeinrichtung, und
- Fig. 4: ein Blockschaltbild des angewendeten Laser-Meßverfahrens, und zwar der Messung der Fluoreszenz.

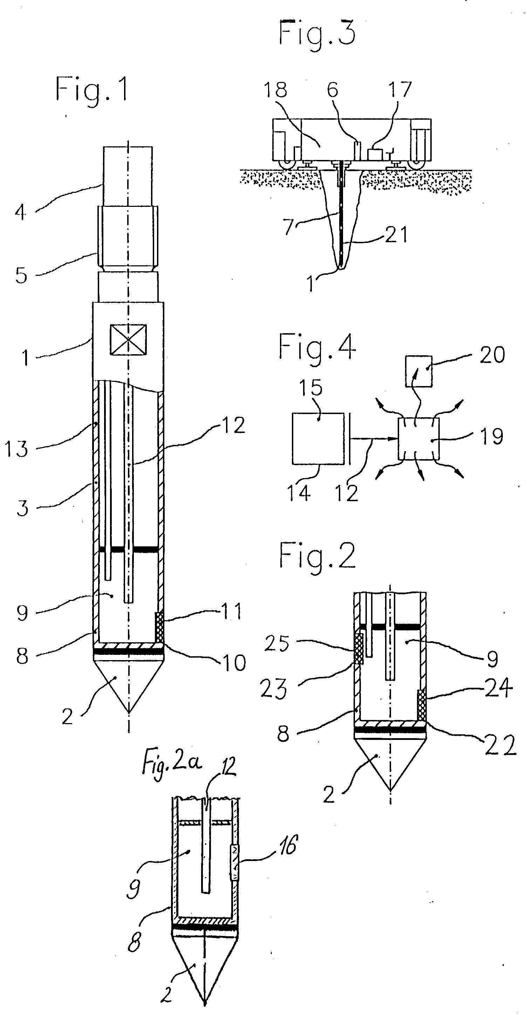

Wie aus Fig. 1 ersichtlich ist, wird die Drucksonde 1 von zwei Hauptteilen gebildet, nämlich von einer kegelig geformten Spitze 2 und einer Zylinderbüchse 3, an deren oberem Ende 4 eine Gewindeausführung 5 zur Befestigung eines an eine Meßeinrichtung 6 anzuschließenden Rohrgestänges 7 (vgl. Fig. 3) ausgebildet ist. Die Zylinderbüchse 3 weist in ihrem unteren Ende 8 eine Meßkammer 9 mit einer Öffnung 10 auf, in die vorteilhaft ein Feinsieb 11 angeordnet ist, um zu gewährleisten, daß als Meßprobe nur Wasser in die Meßkammer eintreten kann. In der Zylinderbüchse 3 und in dem Rohrgestänge 7 ist weiterhin ein Lichtleitkabel 12 und ein Preßluftschlauch 13 angeordnet, die beide in der Meßkammer 9 enden. Das Lichtleitkabel 12 ist Bestandteil einer Lasertechnik 14, (vgl. Fig. 4), die noch weiter unten näher beschrieben wird.

Das Lichtleitkabel 12 wird durch das an die Drucksonde angeschlossene Rohrgestänge 7 bis zu einem in der Meßeinrichtung 6 angeordneten Laser 15 geführt und mit diesem verbunden (vgl. Fig. 3 und 4). Nach der Messung des Grundwassers auf Schadstoffgehalte per Lichtleitkabel 12 wird die Meßkammer 9 durch Preßluft mittels des Preßluftschlauchs 13 gereinigt und somit für die nächste Grundwassermessung vorbereitet.

In Fig. 2 ist ein weiteres Ausführungsbeispiel der Drucksonde 1 dargestellt, wobei in der Meßkammer 9 zwei Öffnungen 22 und 23 angeordnet sind, die das Durchströmen der Meßkammer 9 mit dem zu messenden Grundwasser ermöglichen. In diese Öffnungen 22 und 23 sind vorteilhafterweise Feinsiebe 24 und 25 angeordnet.

Fig. 2a stellt ein weiteres Ausführungsbeispiel der Drucksonde 1 dar, wobei die Öffnung 10 der Meßkammer 9 mit einem durchsichtigen Kristall 16 oder dergleichen verschlossen ist. Dadurch kann das außen an der Meßkammer 9 befindliche Grundwasser gemessen werden. Der Kristall 16 ist für das Laserlicht durchlässig.

Wie aus Fig. 3 ersichtlich wird, ist die Meßeinrichtung 6 mit einem daran angeschlossenen Rechner 17 und einer erforderlichen Vorrichtung 21 zur Erfassung der Bodenstrukturen, in denen sich das auf Schadstoffgehalte zu untersuchende Grundwasser befindet, beispielsweise in einem Fahrzeug 18 über der Bodenmeßstelle angeordnet.

Die Vorrichtung 18 besteht im wesentlichen aus einer in der Zeichnung nicht dargestellten Hydraulik mit der das Rohrgestänge 7 in den Boden beispielsweise bis maximal 30 m eingedrückt werden kann. Hierbei wird die Lagerungsdichte des anstehenden Bodens festgestellt, indem der Spitzendruck und die auf die seitliche Wandung der Drucksonde 1 ausgeübte sogenannte Mantelreibung getrennt aufgenommen wird, so daß hieraus in dem Rechner 17 der Bodenindex direkt ermittelt und ausgewertet werden kann.

In Fig. 4 ist als Blockschaltbild das mit der erfindungsgemäßen Drucksonde 1 vorteilhaft angewendete Meßverfahren schematisch dargestellt. Hierin ist mit 15 der Laser, mit 12 das Lichtleitkabel, mit 19 das fluoreszierende Medium (Grundwasser) und mit 20 der Detektor angegeben.

Für die Drucksonde 1 zum quantitativen Nachweis von Mineralölkohlenwasserstoffen (MKW) und/oder polycyklischen aromatischen Kohlenwasserstoffen (PAK) wird erfindungsgemäß die zeitaufgelöste laserinduzierte Fluoreszenz eingesetzt. Dadurch ergibt sich die Möglichkeit, mit hoher Empfindlichkeit und in kurzer Meßzeit die Konzentration fluoreszierender Schadstoffe zu erfassen. Die Identifikation der MAK und PAK erfolgt anhand des charakteristischen Zeit- und Spektralverhaltens ihrer laserinduzierter Fluoreszenzen.

Da im Mineralöl vor allem die PAK fluoreszieren, ist die Drucksonde 1 auch für den Nachweis von Ölverunreinigungen geeignet. Durch den Einsatz des Lichtleitkabels 12 kann der Meßort von der eigentlichen Meßeinrichtung 6 räumlich entfernt sein. Damit ist eine Tiefensondierung möglich.

Die Auswertung der über die Lasertechnik 14 zur Meßeinrichtung 6 und zum Rechner 17 gelangten Meßsignale erfolgt durch einen Vergleich der im Rechner als Software eingegebenen und gesetzlich tolerierbaren Schadstoffgrößen mit den tatsächlich gemessenen Größen. Damit kann festgestellt werden, ob die Toleranzgröße überschritten wird.

### Bezugszeichenliste

- 1: Drucksonde
- 2: Spitze
- 3: Zylinderbüchse
- 4: oberes Ende der Zylinderbüchse
- 5: Gewindeausführung
- 6: Meßeinrichtung
- 7: Rohrgestänge
- 8: unteres Ende der Zylinderbüchse
- 9: Meßkammer
- 10: Öffnung
- 11: Feinsieb
- 12: Lichtleitkabel
- 13: Preßluftschlauch
- 14: Lasertechnik
- 15: Laser
- 16: Kristall
- 17: Rechner
- 18: Fahrzeug
- 19: absorbierendes Medium (Grundwasser)
- 20: Detektor
- 21: Vorrichtung zur Erfassung der Bodenstrukturen
- 22: Öffnung
- 23: Öffnung
- 24: Feinsieb
- 25: Feinsieb

## Patentansprüche

1. Drucksonde (1) zum quantitativen Nachweis von im Grundwasser vorhandenen Schadstoffen, insbesondere von Mineralölkohlenwasserstoffen und/oder polycyclischen aromatischen Kohlenwasserstoffen, an Ort und Stelle mit einer Meßeinrichtung (6), dadurch gekennzeichnet, daß
a) die Drucksonde (1) zur Ermittlung bodenphysikalischer Parameter einen zusätzlich als Meßkammer (9) ausgebildeten Meßkopf zur Aufnahme einer zu messenden Grundwasserprobe und ein an die Meßeinrichtung (6) angeschlossenes Lichtleitkabel (12) aufweist, und
b) die Meßeinrichtung vorzugsweise aus einer Lasertechnik (14) mit einem angeschlossenen Rechner (17) zur Auswertung besteht, wobei man als Meßverfahren die zeitauflösende laserinduzierte Fluoreszenz einsetzt.

2. Drucksonde nach Anspruch 1, dadurch gekennzeichnet, daß die Drucksonde (1) aus einer kegelig geformten Spitze (2) und einer Zylinderbüchse (3) besteht, an deren oberem Ende (4) eine Gewindeausführung (5) zur Befestigung eines mit der Meßeinrichtung (6) in Verbindung stehenden Rohrgestänges (7) besteht.

3. Drucksonde nach Anspruch 1 oder 2, dadurch gekennzeichnet, daß die Zylinderbüchse (3) in ihrem unteren Ende (8) die Meßkammer (9) mit mindestens einer Öffnung (10) aufweist, in der ein Feinsieb (11) angeordnet ist.

4. Drucksonde nach einem der Ansprüche 1 bis 3, dadurch gekennzeichnet, daß in der Zylinderbüchse (3) und in dem Rohrgestänge (7) ein Lichtleitkabel (12) angeordnet ist, das in der Meßkammer (9) endet.

5. Drucksonde nach mindestens einem der Ansprüche 1 bis 4, dadurch gekennzeichnet, daß das Lichtleitkabel (12) an einer in der Meßeinrichtung (6) angeordneten Lasertechnik (15) angeschlossen ist.

6. Drucksonde nach mindestens einem der Ansprüche 1 bis 5, dadurch gekennzeichnet, daß in der Zylinderbüchse (3) und in dem Rohrgestänge (7) ein Preßluftschlauch (13) angeordnet ist, der in der Meßkammer (9) endet.

7. Drucksonde nach mindestens einem der Ansprüche 1 bis 6, dadurch gekennzeichnet, daß die Zylinderbüchse (3) in ihrem unteren Ende (8) die Meßkammer (9) mit zwei Öffnungen (22 und 23) aufweist, in denen je ein Feinsieb (24 und 25) angeordnet ist.

8. Drucksonde nach mindestens einem der Ansprüche 1 bis 6, dadurch gekennzeichnet, daß die Zylinderbüchse (3) in ihrem unteren Ende (8) die Meßkammer (9) mit einer Öffnung (10) aufweist, in die ein durchsichtiger Kristall (16) angeordnet ist.

9. Drucksonde nach mindestens einem der Ansprüche 1 bis 8, dadurch gekennzeichnet, daß die Meßeinrichtung (6) mit dem Rechner (17) und einer Vorrichtung (21) zur Erfassung der Bodenstrukturen in einem über der Bodenmeßstelle angeordneten Fahrzeug (18) angeordnet ist.
